(19) Europäisches Patentamt
European Patent Office
Office européen des brevets

(11) Veröffentlichungsnummer : **0 053 262**
**B1**

# (12) EUROPÄISCHE PATENTSCHRIFT

(45) Veröffentlichungstag der Patentschrift : **28.12.83**

(51) Int. Cl.$^3$ : **C 07 C 29/00**// C07C69/28, C07C121/34

(21) Anmeldenummer : **81108483.9**

(22) Anmeldetag : **19.10.81**

## (54) Verfahren zur Herstellung von tertiären Alkoholen.

(30) Priorität : **02.12.80 DE 3045378**

(43) Veröffentlichungstag der Anmeldung : **09.06.82 Patentblatt 82/23**

(45) Bekanntmachung des Hinweises auf die Patenterteilung : **28.12.83 Patentblatt 83/52**

(84) Benannte Vertragsstaaten : **BE CH DE FR GB LI**

(56) *Entgegenhaltungen* :
**DE-A- 1 956 018**
**FR-A- 2 003 768**
**US-A- 3 013 038**
**US-A- 3 251 878**
**HOUBEN WEYL: "Methoden der organischen Chemie", Band VI/1a, Teil 2, Alkohole II, 1980, Seiten 910-911 Georg Thieme Verlag Stuttgart, DE**
**HOUBEN WEYL: "Methoden der organischen Chemie", Band VIII, Sauerstoffverbindungen III, 1971, Seite 24, Georg Thieme Verlag, Stuttgart, DE**
**HELVETICA CHIMICA ACTA, Band 36, Fasciculus I-V, 1953, Seiten 13-23 Basel, CH. P. THÜRING et al.: "Oxydation par l'oxygène moléculaire et décarbonylation des aldéhydes"**

(73) Patentinhaber : **BASF Aktiengesellschaft
Carl-Bosch-Strasse 38
D-6700 Ludwigshafen (DE)**

(72) Erfinder : **Merger, Franz, Dr.
Max-Slevogt-Strasse 25
D-6710 Frankenthal (DE)**
Erfinder : **Nestler, Gerhard, Dr.
Van-Leyden-Strasse 17
D-6700 Ludwigshafen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 053 262 B1

## Verfahren zur Herstellung von tertiären Alkoholen

Die Erfindung betrifft ein Verfahren zur Herstellung von tertiären Alkoholen durch Oxidation von tertiären Aldehyden mit Sauerstoff bei einer Temperatur von 60 bis 160 °C ohne Verwendung eines mineralsauren, wasserhaltigen Reaktionsmediums.

Allgemein werden bis jetzt tertiäre Alkohole hauptsächlich entweder aus Olefinen durch Addition von Wasser oder aus Carbonylverbindungen, z. B. Ketone oder Ester, durch Umsetzung mit metallorganischen Verbindungen hergestellt. Außerdem können tertiäre Alkohole auch durch Hydrolyse der entsprechenden Halogenide oder Ester, durch Umsetzung von Epoxiden mit metallorganischen Verbindungen oder durch Oxidation von gesättigten Verbindungen mit kräftigen Oxidationsmitteln, umgesetzt werden (Houben-Weyl, Methoden der organischen Chemie, Band 3, Seiten 285 bis 288).

Ein Nachteil dieser Verfahren ist jedoch die teilweise schwere Zugänglichkeit der Ausgangsprodukte. Die Verfahren befriedigen im Hinblick auf wirtschaftlichen und einfachen Betrieb, hoher Ausbeute an reinem Endstoff, gerade auch im großtechnischen Betrieb, nicht.

Houben-Weyl, Methoden der organischen Chemie, Band VI/1a, Teil 2, Seite 911 (1980), beschreibt die Autoxidation bzw. die Luftoxidation bei Raumtemperatur, wobei das Ausgangsprodukt in Form eines dünnen Filmes vorliegt, von tert. Aldehyden, deren Aldehydgruppe an einem Cyclohexanring des aromatisch-cycloaliphatischen Dehydroabietinals sitzt. Als Oxidationsprodukte treten dabei Gemische von hauptsächlich 2 Hydroperoxiden auf, die nur in geringer Menge (10 bzw. 4 %) die entsprechenden tert. Alkohole enthalten. Zur Herstellung größerer Mengen (30 bzw. 20 %) müssen diese Gemische in einer gesonderten Umsetzung mit Reduktionsmittels reduziert werden (ibid. Seite 911, Zeilen 1-3, 9-11). Entsprechend muß auch ein Formylöstren (ibid, Seiten 910-911) in einer speziellen Reduktionsstufe zum entsprechenden Hydroxy-östren reduziert werden. Entsprechend Caputo et al. (Tetrahedron *29,* (1973) 2047-2051) läuft die Oxidation nach einem Radikalmechanismus ab (ibid, Seite 2049). Während aber üblicherweise die Autoxidation von Aldehyden zu den entsprechenden Carbonsäuren führt, erhält man in den beschriebenen Fällen vorwiegend Hydroperoxid. Nach Caputo et al. (ibid, Seite 2050) ist für dieses anormale Oxidationsverhalten die strukturelle und sterische Anordnung der Aldehydgruppe verantwortlich. Vergleichende Autoxidationsversuche (Tetrahedron *30,* (1974) 963-967) haben nun gezeigt, daß tert. Aldehyde allgemein zur Bildung von anormalen Oxidationsprodukten neigen, wobei tert. Aldehyde mit starren Molekülstrukturen (z. B. Torulosal bzw. Dehydroabietinal) im Gegensatz zu offenkettigen Aldehyden (z. B. Pivalinaldehyd) bevorzugt anormale Oxidationsprodukte liefern (ibid, Seite 965, Tabelle 1). Wie die Tabelle zeigt, führt die Autoxidation von Pivalinaldehyd vorwiegend zur entsprechenden Carbonsäure, d. h. zum normalen Oxidationsprodukt. Die Autoxidation von Pivalinaldehyd wird mit Luft während 7 Tagen bei Raumtemperatur durchgeführt.

Helv. Chim. Acta, Band 36, Seiten 13-23 zeigen die Oxidation von Aldehyden unter Decarboxylierung. DE-A-19 56 018 beschreibt die Oxidation von Isobutyraldehyd mit Sauerstoff in Gegenwart von Oxiden, Hydroxiden bzw. Salzen von Metallen, wobei Aceton und Isopropanol in ähnlicher Ausbeute bzw. mit Aceton als Hauptprodukt erhalten werden. FR-A-20 03 768 zeigt die Umsetzung von Isobutyraldehyd mit Propylen und Sauerstoff in Flüssigphase bei 40 bis 250 °C in einem Gemisch von Propylenoxid, Isopropylalkohol und Aceton. US-A-3 251 878 beschreibt den oxidativen Abbau von Pivalinsäure in essigsaurem Medium mit Sauerstoff in Gegenwart eines Metallsalzes als Katalysator, wobei man ein Gemisch von Methanol, Aceton, t.-Butanol und t.-Butylperoxid erhält. US-A-3 013 038 beschreibt die Herstellung von Gemischen von aliphatischen Alkoholen und Estern durch Umsetzung von Säuren mit Sauerstoff bei mindestens 100 °C in Gegenwart eines fettsauren Kobaltsalzes.

Es wurde nun gefunden, daß man tertiäre Alkohole der Formel

$$R^3 - \underset{R^2}{\overset{R^1}{C}} - OH \qquad (I)$$

worin $R^1$, $R^2$ und $R^3$ gleich oder verschieden sein können und jeweils einen aliphatischen cycloaliphatischen, araliphatischen oder aromatischen Rest bedeuten, vorteilhaft erhält, wenn man tertiäre Aldehyde der Formel

$$R^3 - \underset{R^2}{\overset{R^1}{C}} - CHO \qquad (II)$$

worin $R^1$, $R^2$ und $R^3$ die vorgenannten Bedeutungen besitzen, mit Sauerstoff bei einer Temperatur von 60 bis 160 °C ohne Verwendung eines mineralsäuren, wasserhaltigen Reaktionsmediums umsetzt.

Die Reaktion kann im Falle der Verwendung von 4-Cyano-2,2-dimethylbutyraldehyd durch die folgenden Formeln wiedergegeben werden :

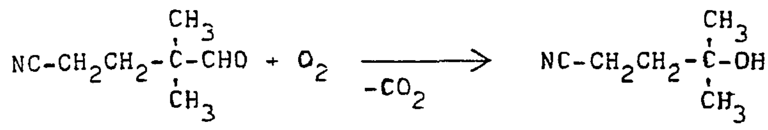

Im Vergleich mit dem Stand der Technik liefert das erfindungsgemäße Verfahren tertiäre Alkohole auf einfacherem und wirtschaftlicherem Wege in besserer Ausbeute und Reinheit. Alle diese vorteilhaften Ergebnisse der Erfindung sind überraschend. So zeigt eine bekannte Umsetzung, daß 4,4-Dimethyl-4-formyl-butansäure bei der Umsetzung mit Sauerstoff lediglich Isocapronsäure liefert (Zeitschrift für Naturforschung, Band 5b (1950), Seite 122), die Umsetzung von 4-Cyano-2,2-dimethyl-butyraldehyd mit Sauerstoff in mineralsaurem, wasserhaltigem Medium 2,2-Dimethylglutarsäure ergibt (DT-PS 16 18 177). Außerdem ist allgemein bekannt, daß die Oxidation von Aldehyden mit Sauerstoff hauptsächlich zu den entsprechenden Carbonsäuren bzw. Anhydriden führt (Houben-Weyl, Methoden der Organischen Chemie, Band 8, Seite 24). Im Hinblick auf Houben-Weyl (loc. cit. Band VI/1a) und Tetrahedron (loc. cit.) war es überraschend, daß gerade tertiäre und offenkettige Aldehyde nach dem erfindungsgemäßen Verfahren die entsprechenden tertiären Alkohole ohne wesentliche Mengen an Carbonsäuren bzw. Hydroperoxiden liefern. Ein zusätzlicher Reduktionsschritt entsprechend Houben-Weyl (loc. cit., Band VI) ist überraschenderweise jedoch nicht erforderlich.

Bevorzugte Ausgangsstoffe II und dementsprechend bevorzugte Endstoffe I sind solche, in deren Formeln $R^1$, $R^2$ und $R^3$ gleich oder verschieden sein können und jeweils einen Alkylrest mit 1 bis 8 Kohlenstoffatomen, insbesondere mit 1 bis 4 Kohlenstoffatomen, eine Cycloalkylgruppe mit 5 bis 8 Kohlenstoffatomen, eine Alkylarylgruppe oder Arylalkylgruppe mit 7 bis 12 Kohlenstoffatomen oder einen Phenylrest bedeuten. Die vorgenannten Reste können noch durch unter den Reaktionsbedingungen inerte Gruppen und/oder Atome, z. B. Alkyl- und Alkoxygruppen mit 1 bis 4 Kohlenstoffatomen, Chloratome, Bromatome, Cyanogruppen, substituiert sein oder gewünschtenfalls können im Falle von aliphatischen $R^1$, $R^2$ und $R^3$ die Kohlenstoffketten durch die Gruppe $-\underset{\underset{O}{\|}}{C}-O-$ unterbrochen werden.

So kommen folgende tertiäre Aldehyde II in Betracht : In α-Stellung durch 3 gleiche oder unterschiedliche Methyl-, Äthyl-, Propyl-, Isopropyl-, Butyl-, sek.-Butyl-, Isobutyl-, tert.-Butyl-, Pentyl-, Cyclopentyl-, Cyclohexyl-, Benzyl-, Phenyl-gruppen substituiertes Acetaldehyd. Bevorzugte Ausgangsstoffe II sind ; 2,2-Dimethyl-3-phenyl-propionaldehyd, 2-Benzyl-2-methyl-butyraldehyd, Acetoxypivalinaldehyd, Isopropylcarbonyloxypivalinaldehyd, 2-Äthyl-2-methyl-3-isopropylcarbonyloxypropionaldehyd, 4-Cyano-2,2-dimethyl-butyraldehyd, 4-Cyano-2-methyl-2-propyl-butyraldehyd oder 4-Cyano-2,2,3-trimethyl-butyraldehyd.

Der Sauerstoff kommt als solcher oder zweckmäßiger in Form von Luft in Betracht. Die Oxidation wird in stöchiometrischer Menge oder im Überschuß an Sauerstoff, zweckmäßig mit 1 bis 10, insbesondere mit 2 bis 8 Mol Sauerstoff, bezogen auf Ausgangsstoff II, vorteilhaft durchgeführt.

Die Umsetzung wird bei einer Temperatur von 60 bis 160 °C, vorzugsweise 70 bis 140 °C, drucklos oder unter Druck, diskontinuierlich oder kontinuierlich durchgeführt. Die Umsetzungen werden vorzugsweise ohne Katalysator durchgeführt. Um höhere Reaktionsgeschwindigkeiten zu erreichen kann es mitunter notwendig sein, in Gegenwart eines Katalysators zu arbeiten. Als Katalysatoren können beispielsweise Schwermetallsalze wie $NiCl_2$, $Ni(OCOCH_3)_2$, $VCl_3$, $CrCl_3$ oder $CeCl_3$ bzw. entsprechende Komplexverbindungen, beispielsweise Tetraimidazol-Ni(II)-chlorid eingesetzt werden. Die Menge des Katalysators ist nicht obligatorisch und liegt allgemein bei 0,01 bis 0,1 Molprozent, bezogen auf das Ausgangsprodukt. Ob ein Katalysator überhaupt notwendig ist und welcher Katalysator zweckmäßig ist, kann leicht jeweils durch einen Vorversuch ermittelt werden.

Die Reaktion kann wie folgt durchgeführt werden : Ein Gemisch von Ausgangsstoff II und Sauerstoff, gewünschtenfalls mit organischem Lösungsmittel und/oder Katalysatoren wird während 2 bis 10 Stunden bei der Reaktionstemperatur gehalten. Dann trennt man den Endstoff in üblicher Weise, z. B. durch fraktionierte Destillation, ab.

Die so herstellbaren tertiären Alkohole sind wertvolle und vielseitige Zwischenprodukte für Farbstoffe, Pharma und Pflanzenschutz und finden beispielsweise bei der Herstellung von Agrochemikalien Verwendung (Jap. Auslegeschrift 14446/68). So kann z. B. Endstoff I in folgender Weise umgesetzt werden :

(Siehe das Schema, Seite 4 f.)

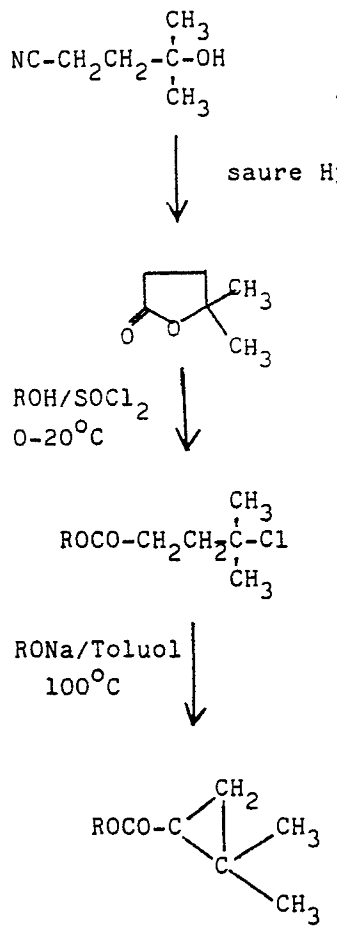


4-Hydroxy-4-methyl-valeronitril

saure Hydrolyse

γ,γ-Dimethyl-butyrolacton

und erhält so wertvolle Zwischenprodukte für Herbizide.

Die in den folgenden Beispielen aufgeführten Teile sind Gewichtsteile.

Beispiel 1

In einem Rührreaktor werden bei 100 °C innerhalb von 5 Stunden 80 Teile Sauerstoff in 172 Teile Isopropylcarbonyloxypivalinaldehyd eingeleitet. Nach Beendigung der Umsetzung wird das Reaktionsgemisch fraktionierend destilliert. Man erhält 118 Teile (74 % Der Theorie) 2-Hydroxy-1-isopropyl-carbonyloxy-2-methylpropan (Kp 48 bis 49 °C/0,4 mbar).

Beispiel 2

Analog Beispiel 1 wird die Umsetzung mit 125 Teilen 4-Cyano-2,2-dimethyl-butyraldehyd durchgeführt. Man erhält 89 Teile (78 % der Theorie) 4-Hydroxy-4-methyl-valeronitril (Kp 112 bis 114 °C/16 mbar).

**Anspruch**

Verfahren zur Herstellung von tertiären Alkoholen der Formel

$$R^3 - \underset{R^2}{\overset{R^1}{C}} - OH \qquad (I)$$

worin $R^1$, $R^2$ und $R^3$ gleich oder verschieden sein können und jeweils einen aliphatischen, cycloaliphatischen, araliphatischen oder aromatischen Rest bedeuten, dadurch gekennzeichnet, daß man tertiäre Aldehyde der Formel

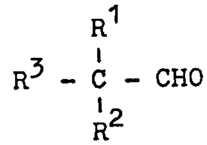

(II)

worin $R^1$, $R^2$ und $R^3$ die vorgenannten Bedeutungen besitzen, mit Sauerstoff bei einer Temperatur von 60 bis 160 °C ohne Verwendung eines mineralsauren, wasserhaltigen Reaktionsmediums umsetzt.

**Claim**

A process for the preparation of a tertiary alcohol of the formula

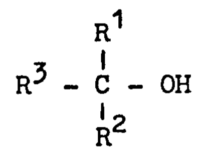

(I)

where $R^1$, $R^2$ and $R^3$ can be identical or different and each is an aliphatic, cycloaliphatic, araliphatic or aromatic radical, wherein a tertiary aldehyde of the formula

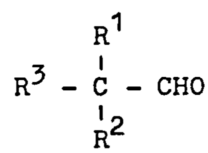

(II)

where $R^1$, $R^2$ and $R^3$ have the above meanings, is reacted with oxygen at 60 to 160 °C without using a reaction medium containing mineral acid and water.

**Revendication**

Procédé de préparation d'alcools tertiaires de la formule

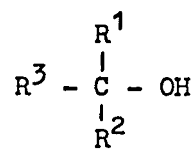

(I)

dans laquelle $R^1$, $R^2$ et $R^3$, qui peuvent être identiques ou différents, désignent chacun un reste aliphatique, cycloaliphatique, araliphatique ou aromatique, caractérisé en ce que l'on fait réagir des aldéhydes tertiaires de la formule

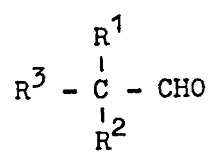

(II)

dans laquelle $R^1$, $R^2$ et $R^3$ possèdent les significations définies, à une température comprise entre 60 et 160 °C avec de l'oxygène, sans utilisation d'un milieu réactionnel aqueux acidifié par un acide minéral.